# EUROPEAN PATENT SPECIFICATION

(11) **EP 3 418 354 B1**
(45) Date of publication and mention of the grant of the patent: **03.06.2020**
(21) Application number: 17382378.2
(22) Date of filing: 20.06.2017
(51) Int. Cl.: C11D 1/62, C11D 3/00, C11D 3/20, C11D 3/50, C11D 11/00, C11D 11/04, C07C 213/06, C07C 213/08

(54) **FABRIC SOFTENER ACTIVE COMPOSITIONS**
WEICHSPÜLERWIRKSTOFFZUSAMMENSETZUNGEN
COMPOSITIONS ACTIVES D'ADOUCISSANTS POUR TISSUS

(43) Date of publication of application: 26.12.2018
(73) Proprietor: KAO CORPORATION, S.A., 08210 Barberà del Vallès, Barcelona (ES)
(72) Inventor: Nogués López, Blanca, 08210 Barberà del Vallès (Barcelona) (ES); Mundó Blanch, Miquel, 08210 Barberà del Vallès (Barcelona) (ES); Pey Gutiérrez, Carmen M., 08210 Barberà del Vallès (Barcelona) (ES); Sobrevias Alabau, Jaume, 08210 Barberà del Vallès (Barcelona) (ES); Vilaret Ferrer, Josep, 08210 Barberà del Vallès (Barcelona) (ES)
(74) Representative: Hoffmann Eitle

(56) References cited:
- EP-A1- 1 136 471
- EP-A2- 0 420 465
- WO-A1-94/19439
- WO-A1-03/022967
- WO-A1-2011/120822
- WO-A1-2012/072368
- US-A- 4 830 771

## Description

### FIELD OF THE INVENTION

The present invention relates to fabric softener active compositions comprising a combination of a quaternary ester ammonium compound and fatty solvents (a fatty acid ester, a fatty acid, a fatty alcohol and mixtures thereof) and methods of making and using the same. The invention also proposes fabric softener compositions, comprising the previously described active compositions, and methods of making and using the same.

### STATE OF THE ART

A fabric softener active composition has to meet several requirements, sometimes difficult to be met simultaneously, to be used in fabric softeners: i) high softening performance, ii) hydrolysis stability in aqueous dispersions with little change in dispersion viscosity, iii) suitable handling and processing in a liquid state, iv) good odour, v) appropriate compatibility with other components including perfumes, vi) ability to contribute to suitable viscosity profiles when to be used: a) in combination with other components of a softener composition and b) at the dilution.

Quaternary ester ammonium compounds, commonly referred to as esterquats, have found broad use as fabric softener actives due to their high softening performance, their biodegradability and reasonably low aquatic toxicity.

Most of the commercially used quaternary ester ammonium compounds are solids. This makes their handling and processing in a pure state difficult: tendency to lump, high viscosity at low melt temperatures, unsatisfactory stability at higher melt temperatures. Use of these compounds in liquid fabric softeners is enhanced by converting them into molten compositions containing from 5 to 25% by weight of a solvent (addition of auxiliary substances is not excluded). The solvent function is to improve the quaternary ester ammonium compounds handling and processing (viscosity reduction in fabric softener active compositions and/or water dispersibility increase from the molten state), providing no benefits in terms of their softening performance though. Commonly used solvents such as ethanol or isopropanol are volatile and flammable substances. Such fabric softener active compositions have a low viscosity, but unfortunately, they have a low flash point of less than 60 °C and therefore require special safety measures when handling and processing and are subject to certain regulatory restrictions.

There are several attempts in the current state of the art aimed to overcome the drawbacks caused by the addition of the cited above flammable solvents.

WO2013126335 A1 proposes fabric softener active compositions which have reduced content of or no added solvents, can flow without having to heat them to very high temperatures that compromise the chemical stability of the product and are able to form stable, low-viscosity liquid fabric softeners. In one embodiment described therein, these fabric softener active compositions comprise at least one quaternary ester ammonium compound and less than 8% added solvent such as isopropanol. In the most preferred embodiment, the fabric softener active compositions contain no solvent. Examples 1-4 show synthesis of quaternary ester ammonium compounds with no solvent added to the reaction product. The viscosity of such fabric softener active compositions is less than 2000 cP at 80°C. They are reported as being easy to handle and process.

EP2553067 B1 discloses fabric softener active compositions having a low content of flammable solvents, a low melt viscosity and high stability in a molten state. These fabric softener active compositions comprise from 65 to 95% of a bis-(-2-hydroxyethyl)-dimethylammonium chloride fatty acid ester, from 2 to 8% of a fatty acid triglyceride (preferably a coconut oil or a hydrogenated coconut oil), and from 3 to 12% of a flammable alcohol selected from ethanol, 1-propanol and 2-propanol. The fabric softener active composition of Example 3 therein is prepared by mixing the powdered esterquat with coconut oil and 2-propanol at a percentage weight ratio of 88:4:8. Melt viscosities measured at 90°C and at shear rates of 1, 10 and 100 s⁻¹ are 262, 236 and 194 cP, respectively. By contrast, melt viscosities of a fabric softener active composition of comparative example 2, consisting of the esterquat and coconut oil at a percentage weight ratio of 94:4, measured at conditions as defined above, are 13200, 9010 and 2290 cP, respectively.

EP2553066 B1 proposes fabric softener active compositions comprising at least 50% by weight of a bis-(2-hydroxypropyl)-dimethylammonium methylsulphate fatty acid ester (preferably from 85 to 95% by weight) and from 0.5 to 5% by weight of a fatty acid (preferably from 2 to 5 % by weight). By adjusting the amount of fatty acid within this range, compositions of the present invention can be made which have low melt viscosities and good storage stability in aqueous dispersions without using any solvent or diluent. In spite of it, the fabric softener active compositions can comprise less than 10% by weight of solvent, having a flash point of less than 20°C. Additionally, they can comprise up to 9.9% by weight of at least one solvent selected from glycerol, ethylene glycol, propylene glycol, dipropylene glycol and C1-C4 alkyl monoethers of ethylene glycol, propylene glycol and dipropylene glycol. Moreover, they can further comprise from 2 to 8% by weight of a fatty acid triglyceride.

EP2553071 B1 discloses fabric softener active compositions having high softening performance and good storage stability in aqueous formulations to which they can be processed to without the use of volatile solvents. These compositions comprise at least 50% by weight of a bis-(2-hydroxypropyl)-dimethylammonium methylsulphate fatty acid ester, and from 0.5 to 5% by weight of a fatty acid. The fabric softener active compositions described therein comprise less than 10% by weight of a flammable solvent. In another preferred embodiment, the fabric softener active composition further comprises from 2 to 8% by weight a fatty acid triglyceride. The compositions obtained are reported to be heat stable.

EP1239024 B1 proposes softener compositions containing a quaternary ammonium salt used as a softener base agent. These softener compositions are reported to be excellent in softening properties, biodegradability and aquatic toxicity. They comprise a cationic surfactant comprising at least one selected from the group consisting of quaternized mono-esteramine (mono-esterquat), quaternized di-esteramine (di-esterquat), quaternized tri-esteramine (tri-esterquat), wherein the ratio of the tri-esterquat to the total amount of mono-esterquat, di-esterquat and tri-esterquat exceeds 50% and the ratio of mono-esterquat to the total amount of mono-esterquat, di-esterquat and tri-esterquat is not more than 10%. The softener compositions further comprise a non-ionic surfactant that is an alkoxylated (ethoxylated, propoxylated, butoxylated) fatty acid ester. Examples 7, 10-15 therein disclose use of ethoxylated hydrogenated tallow fatty acid methyl esters as quaternizing solvents so solutions of ethoxylated hydrogenated tallow fatty acid methyl ester adduct of quaternary ammonium salts are obtained. These solutions are further mixed with water to prepare softener compositions of characteristics as described above.

From the state of the art set forth above, it can be seen that there is still a need for fabric softener active compositions which are able to comply with the requirements imposed on them: i) high softening performance, ii) hydrolysis stability in aqueous dispersions with little change in dispersion viscosity, iii) suitable handling and processing in a liquid state, iv) good odour, and/or v) suitable compatibility with other components including perfumes. Furthermore, there is a need for an improved and more efficient method for obtaining fabric softener active composition comprising fewer steps.

The present invention aims, in particular, at the problem of providing fabric softener active compositions showing low dropping points (preferably below 60°C) in order to allow the handling in a molten state at maximum 70°C, ensuring a good chemical stability, while at the same time having good viscosity values at 70°C so that they can be easily pumped in the molten state. Furthermore, the present invention aims at the further, alternative problem of providing fabric softener active compositions suitable viscosity with a low content or in the absence of flammable solvents. Finally, the present invention also aims at the further, alternative problem of providing fabric softener compositions with improved initial viscosity of their aqueous dispersions and/or improved softening performance properties.

WO 94/19439 A1 describes the use of an ester oil in a fabric softening composition comprising a quaternary ammonium or amine salt fabric softening material to impart ease of iron and/or anti crease benefits to fabric treated with the fabric softening material in which the softening material is a quaternary ammonium or amine compound with two long chain groups connected to the nitrogen via ester links. It does not disclose the presence of a fatty acid ester or a mixture of fatty acid esters derived from a C₁₈-C₂₀ fatty alcohol in the range from 5 to 60% wt.; and it does not disclose the presence of a C₁₈-C₂₀ fatty alcohol in the range from 2 to 12% wt. based on the total weight of the fabric softener active composition.

US 4830771 A describes a process for the preparation of trialkanolamine di(fatty acid) esters in which a trialkanolamine is reacted with a fatty acid in the presence of small amounts of a fatty acid ester. The trialkanolamine fatty acid diesters obtained can be converted into the quaternary ammonium salt by means of standard quaternizing agents, and the resulting products can be employed as fabric conditioners. One object of the invention described therein is to provide a novel method of preparing quaternized trialkanolamine di(fatty acid) esters which results in free-flowing products when in highly concentrated form (85-90% by wt.). Said document teaches the lowering of the pour point by diluting with isopropanol (15 wt.%). The content of fatty acid ester is preferably in the range of 0.1 to 2.0, more preferably 0.2 to 1.0 wt.%. Suitable alcohols from which the fatty acid esters may be derived have 1 to 8, preferably 1 to 6, and more preferably 1 to 3 carbon atoms. The preferred examples relate to tallow fatty acid methyl ester (i.e. derived from a C₁ alcohol).

EP 0420465 A2 describes a method of preparing a fabric softening material which comprises a quaternary ammonium material and at least on C8-28 alkyl or alkenyl group connected to the molecule via an ester linkage, said method comprising the step of reacting a base material with an alkyl or alkenyl group containing material, such that at least one alkyl or alkenyl group is connected to the base material via an ester linkage, wherein the reaction between the base material and the alkyl or alkenyl group containing material is carried out in the presence of an excess of alkyl or alkenyl groups, said excess being effective to lower the pour point of the softener material. A preferred method of preparing softener materials according to the invention involves the reaction of the base-material with a fatty acid, a soap thereof or a C₁-₆ ester thereof, wherein an excess of the fatty acid, its soap or its C₁₋₆ ester is present.

WO 2011/120822 A1 describes a fabric softener active composition, comprising at least 0 % by weight of a bis-(2-hydroxypropyl)-dimethylammonium 5 methylsulphate fatty acid ester having a molar ratio of fatty acid moieties to amine moieties of from 1.5 to 1.99, wherein the average chain length of the fatty acid moieties is from 16 to 18 carbon atoms and the iodine value of the fatty acid moieties,calculated for the free fatty acid, is from 0.5 to 50, and from 0.5 to 5 % by weight fatty acid provides high softening performance and good storage stability in aqueous dispersion and can be handled and processed in a liquid state without addition of a flammable solvent.

WO 03022967 A1 describes a method of thinning a fabric conditioning composition comprising (a) from 7.5 to 80% by weight of a ester-linked quaternary ammonium fabric softening material comprising at least one mono-ester linked component and at least one tri-ester linked component; comprises the step of adding a fatty complexing agent (b) to the composition in an amount such that the weight ratio of the mono-ester linked component of compound (a) to fatty complexing agent (b) is from 2.93:1 to 1:5.

### DEFINITIONS

Fabric softener active composition: A composition comprising a component (a), a component (b), a component (c), and a component (d).

Fabric softener composition: A composition comprising a fabric softener active composition comprising a component (a), a component (b), a component (c) and a component (d), further comprising at least water, wherein the fabric softener active composition is present in an amount from 1 to 30% wt. based on the total weight of the fabric softener composition.

As used herein, the meaning of the term "comprising" encompasses three alternatives, namely "comprising", "consisting of" and "consisting essentially of".

In this specification the non SI-unit cP is used, which may be converted to the SI unit mPa*s as follows: 1 cP = 1 mPa*s.

### SUMMARY OF THE INVENTION

The first object of the present invention is a fabric softener active composition comprising a component (a), a component (b), a component (c) and a component (d), as defined in the claims.

A further object of the present invention is a method for producing a fabric softener active composition comprising a component (a), a component (b), a component (c) and a component (d), as defined in the claims.

Another object of the present invention is a fabric softener composition comprising a fabric softener active composition that comprises a component (a), a component (b), a component (c) and a component (d), further comprising at least water, wherein the fabric softener active composition is present in an amount from 1 to 30% wt. based on the total weight of the fabric softener composition, as defined in the claims.

A method for producing a fabric softener composition as defined in the claims comprising a fabric softener active composition according to the invention is also an object of the present invention.

Another object of the present invention is the use of the fabric softener composition of the invention to soften and condition fabrics.

The textiles or fabrics are conditioned by providing a fabric softener composition comprising a fabric softener active composition according to the invention, contacting one or more fabric articles with the fabric softener composition at one or more points during a laundering process, and allowing the fabric articles to dry or mechanically tumble-drying them.

### DETAILED DESCRIPTION OF THE INVENTION

### FABRIC SOFTENER ACTIVE COMPOSITION

The main object of the present invention is a fabric softener active composition comprising:
- a component (a), said component comprising at least one or more quaternary mono-, di- or tri-ester ammonium compounds;
- a component (b), said component being a fatty acid ester or a mixture of fatty acid esters derived from a C₁₈-C₂₀ fatty alcohol, wherein the component (b) content is in the range from 5 to 60% wt. based on the total weight of the fabric softener active composition;
- a component (c), said component being a fatty acid or a mixture of fatty acids, wherein the component (c) content is higher than 0 and lower than 5, preferably lower than 2% wt. based on the total weight of the fabric softener active composition;
- a component (d), said component being a C₁₈-C₂₀ fatty alcohol, preferably a C₁₈ fatty alcohol; and wherein the component (d) content is in the range from 2 to 12% wt. based on the total weight of the fabric softener active composition, preferably from 2 to 10% wt. based on the total weight of the fabric softener active composition.

Preferably, the fabric softener active composition according to the present invention comprises:
- a component (a), said component comprising at least one or more quaternary mono-, di- or tri-ester ammonium compounds of formula (I1), (I2), (I3), wherein the content of nitrogenated species in the fabric softener active composition is in the range from 40 to 95% wt. , more preferably from 67 to 93% wt. based on the total weight of the fabric softener active composition;
- a component (b), said component being a fatty acid ester or a mixture of fatty acid esters derived from a C₁₈-C₂₀ fatty alcohol, wherein the component (b) content is in the range from 5 to 60% wt. based on the total weight of the fabric softener active composition, preferably from 5 to 40% wt., more preferably from 5 to 30% wt.;
- a component (c), said component being a fatty acid or a mixture of fatty acids, wherein the component (c) content is higher than 0 and lower than 5, preferably lower than 2% wt. based on the total weight of the fabric softener active composition.
   a component (d), said component being a C₁₈-C₂₀ fatty alcohol, preferably a C₁₈ fatty alcohol, wherein the component (d) content is in the range from 2 to 12% wt. based on the total weight of the fabric softener active composition, preferably from 2 to 10% wt. based on the total weight of the fabric softener active composition.

### (a): Quaternary ester ammonium compound:

The fabric softener active composition of the present invention comprises a component (a), said component comprising at least one or more quaternary mono-, di- or tri-ester ammonium compounds (commonly known as mono-esterquat (mono-EQ), di-esterquat(di-EQ), tri-esterquat (tri-EQ)). Preferably, the content of nitrogenated species in the fabric softener active composition is in the range from 40 to 95% wt., more preferably from 67 to 93% wt. based on the total weight of the fabric softener active composition.

In a preferred embodiment of the present invention, said component (a) comprises at least one or more quaternary mono-, di- or tri-ester ammonium compounds of formula (11), (12), (13): 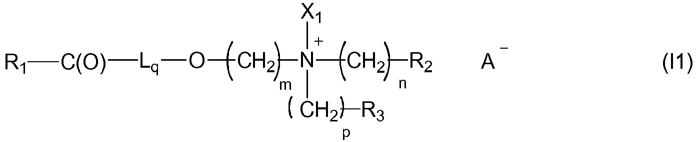 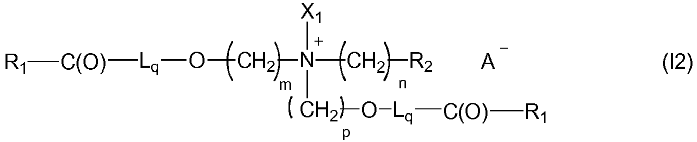 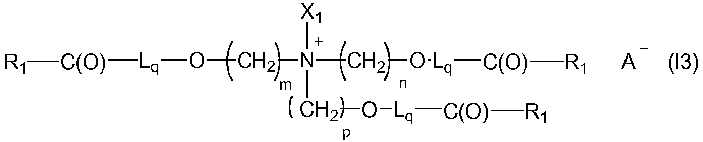 wherein in formulae (11), (12), (13)
R₂ and R₃ each independently represent -H or-OH;
X₁ represents a hydroxyalkyl group containing 1 to 4 carbon atoms, an alkyl group containing 1 to 4 carbon atoms or an alkyl group containing one aromatic group;
R₁ is a linear or branched alkyl containing 5 to 23 carbon atoms or a linear alkenyl group containing 5 to 23 carbon atoms and from 1 to 3 double bonds. In formulae I1, I2 and I3 each R₁ can independently represent the same or different linear or branched alkyl chain;
A⁻ represents an anion;
L represents a -(OCH₂CH₂)ₐ-(OCHR₄CH₂)_{b}- group, wherein R₄ represents an alkyl group containing 1 to 4 carbon atoms, a represents a number within the range of 0 to 20, b represents a number within the range of 0 to 6, and the sum of a+b represents the average alkoxylation degree which corresponds to a number from 0 to 26;
m, n, p each independently represents a number within the range from 1 to 4, q represents a number within the range from 0 to 26.

In a preferred embodiment, said component (a) comprises at least one or more quaternary mono-, di- or tri-ester ammonium compounds of formula (I1), (I2), (I3), wherein the content of nitrogenated species in the fabric softener active composition is in the range from 40 to 95% wt., more preferably from 67 to 93% wt. based on the total weight of the fabric softener active composition.

In a preferred embodiment, the component (a) consists of one or more quaternary mono-, di- or tri-ester ammonium compounds of formula (I1), (I2), (I3).

The quaternary ester ammonium compounds of the invention can be ethoxylated and/or propoxylated, since a and b can be larger than 0. The order of sequence of the ethylene oxide and propylene oxide groups is not critical for the invention.

In the case q is 2 or larger, each L group may be the same or different. Also the (L)q groups contained in the different branches within the compounds of formula (I1), (I2), (I3) may independently represent different meanings.

The sum of a+b preferably represents the average alkoxylation degree which corresponds to a number from 0 to 10, more preferably from 0 to 6, most preferred is 0.

Preferably, X₁ is an alkyl group; more preferably X₁ is a methyl group.

Preferably, A⁻ is selected from a halide, phosphate or alkylsulphate.

Within the present patent application, when a numerical range is indicated, all the individual numbers included in said range are intended to be included. The same shall apply to any other range indicated.

In a particularly preferred embodiment, the component (a) comprises at least one quaternary mono-ester ammonium compound of formula (I1), at least one quaternary di-ester ammonium compound of formula (I2), and at least one quaternary tri-ester ammonium compound of formula (I3), wherein m=n=p=2; R₁-C(O)- is a linear acyl group wherein R₁ is a linear alkyl or a linear alkenyl containing from 11 to 21 carbon atoms, preferably derived from (hydrogenated and/or non-hydrogenated) tallow fatty acid or palm fatty acid; R₂ and R₃ each represent -OH, q is 0 (i.e. the compound is not alkoxylated); X₁ is a methyl group; and A⁻ is selected from a halide, phosphate or alkylsulphate, preferably alkylsulphate.

In another embodiment of the present invention, the component (a) comprises at least one or more quaternary mono-, di- or tri-ester ammonium compounds represented by formula (I1), (I2), (I3) as defined above, wherein R₂ and R₃ independently represent -OH; each m, n, p represents number 2.

The rest of variables have the meanings as indicated above for formula (11), (12), (13).

In another embodiment of the present invention, R₁ is a linear or branched alkyl containing 5 to 23 carbon atoms or a linear alkenyl group containing 5 to 23 carbon atoms and from 1 to 3 double bonds; preferably, the alkyl or alkenyl group contains from 11 to 21 carbon atoms.

As used herein, the term "alkyl" refers to a straight or branched hydrocarbon chain containing from 1 to 23, preferably 5 to 23 carbon atoms.

As used herein, the term "alkenyl" refers to a linear hydrocarbon chain containing from 2 to 23, preferably 5 to 23 carbon atoms and from one to 3 unsaturations.

Linear or branched alkyl or linear alkenyl groups can originate from fatty acids, or methyl esters/triglycerides thereof, are alkyls or alkenyls derived from oils and fats from plants and animals, such as palm, palm kernel, coconut, rapeseed, sunflower, soybean, olive, canola, tall or tallow, possibly totally or partially hydrogenated and purified. Synthetic fatty acids, or methyl esters/triglycerides thereof, such as palmitoleic acid, oleic acid, elaidinic acid, petroselinic acid, linoleic acid, linolenic acid, stearic acid, myristic acid, gadoleic acid, behenic acid and erucic acid, or mixtures thereof, can also be employed in the present invention. Preferably, the linear or branched alkyl or linear alkenyl groups proceed from fatty acids derived from palm oil, coconut oil, tallow and hydrogenated tallow, more preferably from tallow or palm and hydrogenated tallow or palm.

The fatty acid is preferably a C₁₁-C₂₁ acid containing a degree of unsaturation such that the iodine value ("IV") is in the range from 0 to 100, preferably from 10 to 90, more preferably in the range from 15 to 85, most preferably 15 to 55.

The fatty acids employed in the present invention can have a cis to trans isomer ratio from 80:20 to 95:5. Preferably, the trans isomer content of said fatty acid is less than 10%.

As used herein, the term "alkyl group containing one aromatic group" refers to the alkyl group as defined above, substituted by one aromatic group, wherein "aromatic group" refers to an aryl or heteroaryl group.

"Aryl" refers to aromatic ring systems comprising 6 to 14 carbon atoms, more particularly 6 to 10, even more particularly 6 carbon atoms. Examples of aryl groups are phenyl, naphthyl, indenyl, fenanthryl or anthracyl radical, preferably phenyl or naphthyl radical. Said aryl radical may be optionally substituted by one or more substituents such as hydroxy, mercapto, halo, alkyl, phenyl, alkoxy, haloalkyl, nitro, cyano, dialkylamino, aminoalkyl, acyl and alkoxycarbonyl, as defined herein.

"Alkoxy" refers to an alkyl group as defined above bonded to an oxygen atom (R-O-).

Examples of halogen atoms are Br, Cl, I and F.

The term "heteroaryl" means a monocyclic- or polycyclic aromatic ring comprising carbon atoms, hydrogen atoms, and one or more heteroatoms, preferably, 1 to 3 heteroatoms, independently selected from nitrogen, oxygen, and sulfur. The heteroaryl group has 3 to 15 members and preferably 4 to 8 members. Illustrative examples of heteroaryl groups include, but are not limited to, pyridinyl, pyridazinyl, pyrimidyl, pyrazyl, triazinyl, pyrrolyl, pyrazolyl, imidazolyl, (1,2,3,)- and (1,2,4)-triazolyl, pyrazinyl, pyrimidinyl, tetrazolyl, furyl, thienyl, isoxazolyl, thiazolyl, phenyl, isoxazolyl, and oxazolyl. A heteroaryl group can be unsubstituted or substituted with one or two suitable substituents such as hydroxy, mercapto, halo, alkyl, phenyl, alkoxy, haloalkyl, nitro, cyano, dialkylamino, aminoalkyl, acyl and alkoxycarbonyl, as defined herein. Preferably, a heteroaryl group is a monocyclic ring, wherein the ring comprises 2 to 5 carbon atoms and 1 to 3 heteroatoms.

### Preparation of quaternary ester ammonium compound:

The component (a) comprises at least one or more quaternary mono-, di- or tri-ester ammonium compounds according to the invention. The component (a) can be prepared by i) esterification, reacting a fat source, preferably a fatty acid or a methyl ester/triglyceride thereof, with an alkanolamine (for example, but not limited to, triethanolamine, methyldiethanolamine or dimethylethanolamine) to obtain a mixture containing an esteramine, and ii) subsequently quaternizing the mixture with an alkylating agent.

### i) Esterification step:

It is preferred that the fat source employed in the esterification step is a fatty acid or a mixture of fatty acids. In case a fatty acid methyl ester or a fatty acid triglyceride is used, the transesterification conditions are those described in the state of the art.

The reaction between the fatty acid and the alkanolamine is an esterification which leads to the formation of an esteramine or a mixture of esteramines, and it may be conducted in a known way, as described for example in document ES-A-2021900. Preferably the esterification reaction is carried out at a temperature between 150 and 200°C, for a period of 2-10 hours, preferably at a reduced pressure of about 5 to 200 mbar and in the presence of one of the catalysts known for the esterification, such as hypophosphorous acid or paratoluenesulfonic acid, and also in the presence of any of the usual stabilizers and antioxidants such as tocopherols, BHT, BHA, etc.

In an embodiment of the present invention, a fatty alcohol or a mixture of fatty alcohols is additionally added to the system in the esterification step. A part of the fatty acid present in the system may react with a fatty alcohol resulting in a fatty acid fatty alcohol ester, as a further product of the esterification step, formed in addition to the esteramine/s. Suitable fatty alcohol is a C18-C20 alcohol or alkoxylated alcohol, or polyol, preferably a C₁₈ alcohol.

In an embodiment of the present invention, the fatty alcohol has the following formula (I4)

R₅-O-(CH₂CH₂O)ₛ-(CH₂CHR₆O)ₜ-H (I4)

Wherein R₅ represents an alkyl or alkenyl group, linear or branched containing 18 to 20 carbon atoms; R₆ represents an alkyl group containing 1 to 4 carbon atoms; s represents a number within the range of 0 to 20, t represents a number within the range of 0 to 20, and the sum of s + t represents the average alkoxylation degree which corresponds to a number from 0 to 40, preferably from 0 to 20, more preferably from 0 to 15.
The fatty alcohol can be ethoxylated and/or propoxylated, since s and t can be larger than 0. The order of sequence of the ethylene oxide and propylene oxide groups is not critical for the invention.

In an embodiment of the present invention, a C₁₈ fatty alcohol is additionally added to the system in the esterification step. A part of the fatty acid present in the system may react with the C₁₈ fatty alcohol resulting in a fatty acid fatty alcohol ester, as a further product of the esterification step, formed in addition to the esteramine/s.

In another embodiment of the present invention, an alkoxylated C₁₈ fatty alcohol is additionally added to the system in the esterification step, wherein the alkoxylated C₁₈ fatty alcohol can be ethoxylated and/ or propoxylated. A part of the fatty acid present in the system may react with the alkoxylated C₁₈ fatty alcohol resulting in a fatty acid fatty alcohol ester, as a further product of the esterification step, formed in addition to the esteramine/s.

The molar ratio of fatty acid to alkanolamine is from 1.4:1 to 2.5:1, preferably from 1.6:1 to 2.2:1.

The product resulting from the esterification reaction comprises at least one or more mono-, di- and tri-esters of fatty acids. The product may also contain free alkanolamine, free fatty acid, fatty acid fatty alcohol ester and free fatty alcohol. The progress of the reaction may be monitored by non-aqueous potentiometric titration with KOH.

### ii) Quaternization step:

The quaternization of the esterification reaction product of alkanolamine with the fatty acid is conducted in a known way, as described for example in WO-A-9101295. Preferred alkylating agents include, but are not limited to, methyl chloride, dimethyl sulphate or mixtures thereof.

The quaternization may take place in bulk or in solvent, at temperatures ranging from 40 to 90°C. If an added solvent is employed, then the starting materials and/or product must be soluble in the solvent to the extent necessary for the reaction (possible solvents can be the same solvents as used as component (b), component (c) and component (d) as defined below).

The composition that results from the quaternization process comprises quaternised ester compounds having one (monoesterquat), two (diesterquat) or three (triesterquat) ester groups. The product may also contain quaternised alkanolamine, unreacted esteramine, unreacted fatty acid, fatty acid alkyl ester such as fatty acid methyl ester or fatty acid ethyl ester, as well as fatty alkyl methyl ether.

In an embodiment of the present invention the content of fatty acid alkyl ester, preferably a fatty acid methyl ester or a fatty acid ethyl ester is in the range from 0 to 5% wt. based on the total weight of the fabric softener active composition, preferably in the range from 0 to 3% wt. based on the total weight of the fabric softener active composition, more preferably in the range from 0 to 2% wt. based on the total weight of the fabric softener active composition.

In an embodiment of the present invention, the component (a), said component comprising at least one or more quaternary mono-, di- or tri-ester ammonium compounds of formula (I1), (I2), (I3), is obtained from an esteramine mixture obtained by esterification of triethanolamine and tallow and/or hydrogenated tallow fatty acid and/or palm fatty acid followed by quaternization.

The quaternization reaction may take place in a degree from 60 to 95% of the totality of the reaction.

Preparation of the component (a) is carried out under conditions according to the person skilled in the art to obtain a mixture of at least one or more quaternary mono-, di- or tri-ester ammonium compounds of formula (I1), (I2), (I3).

In an embodiment of the present invention, the component (a) comprises at least one or more quaternary mono-, di- or tri-ester ammonium compounds of formula (I1), (I2), (I3), wherein m=n=p; R₁-C(O)- is a linear acyl group wherein R₁ is an alkyl or alkenyl group containing from 11 to 21 carbon atoms, preferably derived from (hydrogenated or non-hydrogenated) tallow fatty acid or palm fatty acid; R₂ and R₃ each represent -OH, q is 0 (i.e. the compound is not ethoxylated); X₁ is a methyl group; and A⁻ is selected from a halide, phosphate or alkylsulphate, preferably alkylsulphate. Such compound may be produced by esterifying (hydrogenated and/or non-hydrogenated) tallow fatty acid or palm fatty acid and triethanolamine, wherein the ratio of tallow fatty acid or palm fatty acid to triethanolamine is from 1.6:1 to 2.2:1, and subsequently methylating the esteramine obtained thereby.

### (b): Fatty acid ester

The fabric softener active composition of the present invention comprises a component (b), said component being a fatty acid ester or a mixture of fatty acid esters derived from a C₁₈-C₂₀ fatty alcohol, wherein the component (b) content is in the range from 5 to 60% wt. based on the total weight of the fabric softener active composition, preferably from 5 to 40% wt., more preferably from 5 to 30% wt.

The component (b) present in the fabric softener active composition is intentionally added in the esterification step, after the esterification step, in the quaternization step, or after the quaternization step and/or generated in situ in the esterification step or in the quaternization step.

In one embodiment of the present invention, the component (b) present in the fabric softener active composition is obtained in the esterification step by the reaction between the fatty acid or the mixture of fatty acids and a fatty alcohol or a mixture of fatty alcohols additionally added into the system.

In another embodiment of the present invention, the component (b) present in the fabric softener active composition is added to the system after the esterification step has finished, and can act as a solvent for the quaternization step.

In another embodiment of the present invention, the component (b) present in the fabric softener active composition is added to the component (a) after the quaternization step as an additive.

Yet in another embodiment of the present invention, the component (b) present in the fabric softener active composition corresponds to the combination of the previously described embodiments.

In one preferred embodiment of the invention, the component (b) has the following formula (I5):

*R*₇ - *COO* - *R*₈ (I5)

wherein
R₇ represents a fatty acid moiety being a linear or branched alkyl containing 5 to 23 carbon atoms or a linear alkenyl group containing 5 to 23 carbon atoms and from 1 to 3 double bonds. Preferably, the alkyl or alkenyl group contains from 11 to 21 carbon atoms. Preferably, the alkyl or alkenyl group proceeds from fatty acids derived from palm oil, coconut oil, tallow and hydrogenated tallow, more preferably from tallow or palm and hydrogenated tallow or palm.
R₈ represents an alkyl or alkenyl group derived from a linear or branched alcohol containing 18 to 20 carbon atoms, or represents an alkyl or alkenyl group derived from a linear or branched alkoxylated alcohol represented by formula (I6)

   R₉-O-(CH₂CH₂O)ᵤ-(CH₂CHR₁₀O)ᵥ- (I6)

   Wherein Rg represents an alkyl or alkenyl group, linear or branched containing 18 to 20 carbon atoms; R₁₀ represents an alkyl group containing 1 to 4 carbon atoms; u represents a number within the range of 0 to 20, v represents a number within the range of 0 to 20, and the sum of u+v represents the average alkoxylation degree wich corresponds to a number from 0 to 40, preferably from 0 to 20, more preferably from 0 to 15.

The group derived from the fatty alcohol can be ethoxylated and/or propoxylated, since u and v can be larger than 0. The order of sequence of the ethylene oxide and propylene oxide groups is not critical for the invention.

In a preferred embodiment of the present invention, R₈ represents an alkyl or alkenyl group derived from a linear or branched, possibly alkoxylated (ethoxylated, propoxylated), alcohol containing 18 carbon atoms.

In another embodiment of the present invention, the component (b) is derived from: i) polyols, such as glycerol, sorbitol, pentaerythritol, etc. or ii) low or polymeric glycols, such as ethylene glycol, propylene glycol, dipropylene glycol, polyethylene glycol, polypropylene glycol, etc.

In another embodiment of the present invention, the fatty acid moieties in the component (b) and the component (a) are derived from the same fatty acid or mixture of fatty acids.
In the present invention, the component (b) is a fatty acid ester or a mixture of fatty acid esters derived from a C₁₈-C₂₀ fatty alcohol or a mixture of C₁₈-C₂₀ fatty alcohols.

In another preferred embodiment of the present invention, the component (b) is a fatty acid ester or a mixture of fatty acid esters derived from a C₁₈ fatty alcohol.

In another preferred embodiment of the present invention, the component (b) is a fatty acid ester or a mixture of fatty acid esters derived from an alkoxylated C₁₈ fatty alcohol.

### (c): Fatty acid

The fabric softener active composition of the present invention comprises a component (c), said component being a fatty acid or a mixture of fatty acids, wherein the content of component (c) is higher than 0 and lower than 5% wt. based on the total weight of the fabric softener active composition, preferably lower than 2% wt., even more preferably lower than 0.5% wt.

The component (c) present in the fabric softener active composition is intentionally added in the esterification step, after the esterification step, in the quaternization step, or after the quaternization step and/or accounts for an unreacted material.

In one embodiment of the present invention, the component (c) present in the fabric softener active composition corresponds to a free or unreacted fatty acid obtained after the esterification step which has not reacted with alkylating agent in the quaternization step to form a fatty acid methyl ester.

In another embodiment of the present invention, the component (c) present in the fabric softener active composition corresponds to a free or unreacted fatty acid obtained after the esterification step, which has not reacted with fatty alcohol to form a fatty acid fatty alcohol ester.

In another embodiment of the present invention, the component (c) present in the fabric softener active composition corresponds to a fatty acid or a mixture of fatty acids added to the esterification product, before the quaternization step, and which has not reacted with alkylating agent in the quaternization step to result in a fatty acid methyl ester.

In another embodiment of the present invention, the component (c) present in the fabric softener active composition is added to the component (a) after the quaternization step as an additive.
Yet in another embodiment of the present invention, the component (c) present in the fabric softener active composition corresponds to the combination of the previously described embodiments.

Suitable C₆-C₂₂ fatty acids are those obtained from vegetable and animal oils and fats such those obtained from castor oil, coconut oil, corn oil, mustard oil, olive oil, palm oil, peanut oil, rapeseed oil, sunflower oil, soybean oil, tall oil, tallow, eventually totally or partially hydrogenated, as well as purified or synthetic fatty acids, like caproic acid, caprylic acid, capric acid, isotridecanoic acid, lauric acid, myristic acid, palmitic acid, palmoleic acid, stearic acid, isostearic acid, 2-ethylhexanoic acid, oleic acid, elaidinic acid, petroselenic acid, linoleic acid, linolenic acid, eleostearic acid, ricinoleic acid, arachidic acid, gadoleic acid, behenic acid, erucic acid, or their technical-grade mixtures.

In another embodiment of the present invention, the component (c) is a C₈-C₂₀ fatty acid or a mixture of C₈-C₂₀ fatty acids, preferably a
C₁₂-C₂₀ fatty acid or a mixture of C₁₂-C₂₀ fatty acids.

In another embodiment of the present invention, the component (c) and the component (a) are derived from the same fatty acid or mixture of fatty acids.

The fatty acid is preferably a C₈-C₂₀ acid, more preferably a C₁₂-C₂₀ acid containing a degree of unsaturation such that the iodine value ("IV") is in the range 0-90, preferably 10-90, more preferably in the range 15-85, most preferably 15-55.

### (d): Fatty alcohol

The fabric softener active composition of the present invention comprises a component (d), said component being a C₁₈-C₂₀ fatty alcohol or a mixture of a C₁₈-C₂₀ fatty alcohols, wherein the component (d) content is in the range from 2 to 12% wt. based on the total weight of the fabric softener active composition, preferably from 2 to 10% wt., more preferably from 2.1 to 4.9% wt., more preferably from 5.1 to 9.9% wt., further preferably more than 2.1 and less than 4.9% wt., and further preferably more than 5.1 and less than 9.9% wt.

The component (d) present in the fabric softener active composition is intentionally added in the esterification step, after the esterification step, in the quaternization step, or after the quaternization step and/or accounts for an unreacted material.
In one embodiment of the present invention, the component (d) present in the fabric softener active composition corresponds to a free or unreacted fatty alcohol that has not reacted with a fatty acid in the esterification step to form a fatty acid fatty alcohol ester.

In another embodiment of the present invention, the component (d) present in the fabric softener active composition corresponds to a fatty alcohol or a mixture of fatty alcohols added to the esterification product as a quaternizing solvent.

In another embodiment of the present invention, the component (d) present in the fabric softener active composition is added to the component (a) after the quaternization step as an additive.

Yet in another embodiment of the present invention, the component (d) present in the fabric softener active composition corresponds to the combination of the previously described embodiments.

Suitable fatty alcohol is a C₁₈-C₂₀ alcohol or alkoxylated (ethoxylated, propoxylated, butoxylated) alcohol, or polyol, preferably a C₁₈ alcohol of formula I7:

R₁₁-O-(CH₂CH₂O)ₚ-(CH₂CHR₁₂O)_{q}-H (I7)

Wherein R₁₁ represents an alkyl or alkenyl group, linear or branched containing 18 to 20 carbon atoms; R12 represents an alkyl group containing 1 to 4 carbon atoms; p represents a number within the range of 0 to 20, q represents a number within the range of 0 to 20, and the sum of p + q represents the average alkoxylation degree which corresponds to a number from 0 to 40, preferably from 0 to 20, more preferably from 0 to 15.
The fatty alcohol can be ethoxylated and/or propoxylated, since p and q can be larger than 0. The order of sequence of the ethylene oxide and propylene oxide groups is not critical for the invention.

In an embodiment of the present invention, the component (d) corresponds to a C₁₈ fatty alcohol and/or a C₁₈ alkoxylated (ethoxylated and/or propoxylated) fatty alcohol.

The fabric softener active composition according to the present invention may contain further components.

### (e): Solvent

The present invention may further comprise a component (e), said component being a solvent. In a preferred embodiment, the component (e) content is 0% wt. or more and lower than 8% wt. based on the total weight of the fabric softener active composition, preferably 0% wt. or more and lower than 6% wt., more preferably 0% wt. or more and lower than 5% wt.

In the most preferred embodiment, the fabric softener active composition comprises essentially no solvent. The fabric softener active composition does not require the presence of a solvent to comply with the purpose of the invention.

Solvents useful in the present technology include flammable liquids of flash point equal to or lower than 40°C selected from the following list: methanol, ethanol, 1-propanol, 2-propanol, 1-butanol, 2-butanol, hexane, heptane, and combinations thereof. Preferably, the solvent is ethanol or 2-propanol and most preferably 2-propanol.

Other suitable solvents for use in the present invention include ethylene glycol, trimethylene glycol, tetramethylene glycol, pentamethylene glycol, hexamethylene glycol, diethylene glycol, triethylene glycol, propylene glycol, dipropylene glycol and C₁-C₄ alkyl monoethers of ethylene glycol, propylene glycol, and dipropylene glycol, sorbitol, alkane diols such as 1,2-propanediol, 1,3-propanediol, 2,3-butanediol, 1,4-butanediol, 1,3-butanediol, 1,5-pentanediol, and 1,6 hexanediol; phenylethyl alcohol, 2-methyl- 1,3-propanediol, hexylene glycol, sorbitol, polyethylene glycols, 1,2-hexanediol, 1,2-pentanediol, 1,2-butanediol, 1,4-cyclohexanedimethanol, pinacol, 2,4-dimethyl- 2,4-pentanediol, 2,2,4-trimethyl-I,3-pentanediol (and ethoxylates), 2-ethyl-I,3-hexanediol, phenoxyethanol (and ethoxylates), glycol ethers, butyl carbitol, dipropylene glycol n-butyl ether, or combinations thereof.

A method to obtain a fabric softener active composition according to the present invention comprises:
i) an esterification step, wherein a fatty acid, a methyl ester or a triglyceride thereof is reacted with an alkanolamine to obtain a mixture containing an esteramine; and
ii) a quaternization step, wherein the mixture obtained after the esterification step is reacted with an alkylating agent.

Preferably, a C₁₈-C₂₀ fatty alcohol, more preferably a C₁₈ fatty alcohol, is added in the esterification step, after the esterification step, in the quaternization step or after the quaternization step. Even more preferably, a fatty acid, a methyl ester or a triglyceride thereof is added in the quaternization step or after the quaternization step.

The term "added in the esterification/quaternization step" as used herein refers to addition of the respective component to the esterification/quaternization reaction mixture either prior to or in course of the esterification/quaternization reaction.

The method to obtain a fabric softener active composition according to the present invention is characterized in that the component (b) present in the fabric softener active composition is intentionally added in the esterification step, after the esterification step, in the quaternization step or after the quaternization step and/or generated in situ in the esterification step or in the quaternization step. Preferably, the method to obtain a fabric softener active composition according to the present invention may be further characterized in that the component (c) present in the fabric softener active composition is intentionally added in the esterification step, after the esterification step, in the quaternization step or after the quaternization step and/or corresponds to an unreacted material. Even more preferably, the method to obtain a fabric softener active composition according to the present invention may be further characterized in that the component (d) present in the fabric softener active composition is intentionally added in the esterification step, after the esterification step, in the quaternization step or after the quaternization step and/or corresponds to an unreacted material.

Preferably, the fabric softener active composition of the present invention comprises:
- a component (a), said component comprising at least one or more quaternary mono-, di- or tri-ester ammonium compounds of formula (I1), (I2), (I3), wherein the content of nitrogenated species in the fabric softener active composition is in the range from 40 to 95% wt., more preferably from 67 to 93% wt. based on the total weight of the fabric softener active composition;
- a component (b), said component being a fatty acid ester or a mixture of fatty acid esters derived from a C₁₈-C₂₀ fatty alcohol, wherein the component (b) content is in the range from 5 to 60% wt. based on the total weight of the fabric softener active composition, preferably from 5 to 40% wt., more preferably from 5 to 30% wt.;
- a component (c), said component being a fatty acid or a mixture of fatty acids, wherein the component (c) content is higher than 0 and lower than 5% wt. based on the total weight of the fabric softener active composition, preferably lower than 2% wt. based on the total weight of the fabric softener active composition.
- a component (d), said component being a C₁₈-C₂₀ fatty alcohol, preferably a C₁₈ fatty alcohol, wherein the component (d) content is in the range from 2 to 12% wt. based on the total weight of the fabric softener active composition, preferably from 2 to 10 % wt. based on the total weight of the fabric softener active composition.

In one embodiment of the present invention, the component (a) comprises at least one quaternary mono-ester ammonium compound of formula (I1), at least one quaternary di-ester ammonium compound of formula (12), and at least one quaternary tri-ester ammonium compound of formula (13), wherein m=n=p=2; R₁-C(O)- is a linear acyl group wherein R₁ is a linear alkyl or alkenyl containing from 11 to 21 carbon atoms, preferably derived from (hydrogenated or non-hydrogenated) tallow fatty acid or palm fatty acid; R₂ and R₃ each represent -OH, q is 0 (i.e. the compound is not alkoxylated); X₁, is a methyl group; and A⁻ is selected from a halide, phosphate or alkylsulphate, preferably alkylsulphate. Such a compound may be produced by esterifying tallow fatty acid and triethanolamine, wherein the molar ratio of tallow fatty acid to alkanolamine is 1.4-2.5, preferably 1.6-2.2, and subsequently methylating the esteramine obtained thereby, wherein the degree of quaternization is from 25 to 95%.

In another embodiment of the present invention, the component (b) is a C₁₈-₂₀ fatty acid ester, wherein the fatty acid source is preferably palm oil, coconut oil, tallow and hydrogenated tallow, more preferably palm oil and/or tallow and hydrogenated tallow.

In another embodiment of the present invention, the component (c) is preferably a C₁₂-C₂₀ fatty acid containing a degree of unsaturation such that the iodine value ("IV") is in the range 15-55.

In another embodiment of the present invention, the component (d) is a C₁₈-C₂₀ fatty alcohol. In another embodiment of the present invention, the component (d) is an alkoxylated (ethoxylated and/or propoxylated) C₁₈-C₂₀ fatty alcohol.

In another embodiment of the present invention, the fabric softener active composition further comprises a component (e), said component being a solvent, wherein the solvent content is higher than 0% and lower than 8% wt. based on the total weight of the fabric softener active composition, preferably lower than 6% wt., more preferably lower than 5% wt.

In another embodiment of the present invention, the component (e) is chosen from ethanol, 1-propanol and 2-propanol. The component (d) is preferably ethanol or 2-propanol and most preferably 2-propanol.

Yet in another embodiment of the present invention, the component (e) can further comprise glycols, preferably propylene glycol.

In an embodiment of the present invention, the fabric softener active composition comprises, in the indicated amounts expressed as percentage by weight with respect to the total weight of the composition:
- at least one or more quaternary mono-, di- or tri-ester ammonium compounds of the component (a),
- 5 to 60% of the component (b),
- higher than 0 and lower than 5% of the component (c),
- 2 to 12% of the component (d).
In another embodiment of the invention, the fabric softener active composition comprises, in the indicated amounts expressed as percentage by weight with respect to the total weight of the composition:
- at least one or more quaternary mono-, di- or tri-ester ammonium compounds of the component (a),
- 5 to 60% of the component (b),
- higher than 0 and lower than 5% % of the component (c),
- 2 to 12% of the component (d),
- 0 to 8% of the component (e).
In a particularly preferred embodiment of the present invention, the fabric softener active composition comprises no component (e).
In another embodiment of the present invention, the sum content of the component (a), the component (b) and the component (c) is in the range from 5 to 80% wt. based on the total weight of the fabric softener active composition, preferably from 10 to 60% wt., more preferably from 20 to 40% wt. based on the total weight of the fabric softener active composition. Additionally, the weight ratio (b)/(c) of the component (b), and the component (c) is equal to or higher than 45/55, preferably in the range from 45/55 to 99.5/0.5, whereas the weight ratio (b)/(d) of the component (b), and the component (d) is in the range from 50/50 to 95/5.
In an embodiment of the present invention, the fabric softener active composition consists of components (a), (b), (c) and (d). In another embodiment of the present invention, the fabric softener active composition consists of components (a), (b), (c), (d) and (e).

In an embodiment of the present invention, the fabric softener active composition contains from 40 to 95% wt., more preferably from 67 to 93% wt. of nitrogenated species based on the total weight of the fabric softener active composition.

The fabric softener active composition can be used to soften fabrics by treating the fabric with the composition. This can be done in a dryer when using a dryer sheet impregnated with the fabric softening active composition.

### FABRIC SOFTENER COMPOSITION

Another object of the present invention is a fabric softener composition comprising a fabric softener active composition that comprises a component (a), a component (b), a component (c), and a component (d), further comprising at least water, wherein the fabric softener active composition is present in an amount from 1 to 30% wt. based on the total weight of the fabric softener composition, more preferably from 1.5 to 25% wt., most preferably from 2 to 20% wt.

In one embodiment of the present invention, the fabric softener composition further comprises optional components. In referring to the optional components, without this having to be regarded as an exhaustive description of all possibilities, which, on the other hand, are well known to the person skilled in the art, the following may be mentioned:
a) other products that enhance the performance of the softener compositions, such as silicones, amine oxides, anionic surfactants, such as lauryl ether sulphate or lauryl sulphate, amphoteric surfactants, such as cocoamidopropyl betaine or alkyl betaines, sulphosuccinates, polyglucoside derivatives, etc.
b) stabilising products, such as salts of amines having a short chain, which are quaternised or non-quaternised, for example of triethanolamine, N-methyldiethanolamine, etc., and also non-ionic surfactants, such as ethoxylated fatty alcohols, ethoxylated fatty amines.
c) products that improve viscosity control, such as inorganic salts, for example, calcium chloride, magnesium chloride, calcium sulphate, sodium chloride, etc.; products which can be used to reduce viscosity in concentrated compositions, such as compounds of the glycol type, for example, ethylene glycol, dipropylene glycol, polyglycols, etc.; thickening agents for diluted compositions, such as polymers, suitable polymers are water soluble or dispersible, preferably the polymers are cationic. Suitable cationic polymeric materials include cationic guar polymers, cationic cellulose derivatives, cationic potato starch, cationic polyacrylamides. Specially suitable are cross-linked water swellable cationic polymers. Those described polymers may also act as deposition aids.
d) components for adjusting the pH, which is from 2.0 to 6.0, preferably from 2.5 to 4.0, such as any type of inorganic and/or organic acid, for example hydrochloric, sulphuric, phosphoric, citric acid etc.
e) agents that improve soil release, such as the known polymers or copolymers based on terephthalates.
f) preservatives, such as bactericides, for example, 1,2-benzisothiazolin-3-one, 5-chloro-2-methyl-4-isothiazolin-3-one and 2-methyl-4-isothiazolin-3-one, or their combinations, 2-bromo-2-nitropropane-1,3-diol, etc.
g) other products such as antioxidants, colouring agents, perfumes, germicides, fungicides, anti-corrosive agents, anti-crease agents, opacifiers, optical brighteners, pearl lustre agents, etc.

In a preferred embodiment of the present invention, the fabric softener composition comprises a perfume or a perfume microcapsule, wherein the perfume content is lower than 5% wt. based on the total weight of the fabric softener composition, preferably lower than 3% wt., more preferably lower than 2% wt.

In a particularly preferred embodiment of the present invention, the fabric softener composition comprises:
a) from 0 to 2% of an electrolyte concentration aid, preferably from 0.01 to 1%, more preferably from 0.02 to 0.5%; and/or
b) from 0.01 to 3% of a thickening polymer, preferably from 0.02 to 1%, more preferably from 0.05 to 0.5%; and/or
c) from 0.01 to 5% of a perfume, alternatively from 0.1 to 4% or from 0.2 to 4% of a neat perfume and optionally from 0.01 to 3%, preferably from 0.1 to 2%, more preferably from 0.3 to 1%, of a perfume microcapsule.

### PREPARATION OF FABRIC SOFTENER COMPOSITION

The fabric softener composition of the present invention can be obtained following a conventional process of mixing the different components, well known by any skilled person. For example, the different components can be mixed in the molten state, added to the water and stirred to obtain a homogeneous dispersion and then cooled down. In a preferred process of obtention, electrolytes and/or polymers, if present in the composition, are added to the water previous to the dispersion of the fabric softener active composition, or once the fabric softener active composition is dispersed in water. Perfume is preferably added once the fabric softener active composition is dispersed in water and the blend is cooled down.

### METHOD FOR CONDITIONING TEXTILES

The fabric softener composition according to the invention can be used in both a so-called non-rinse and a so-called rinse process, where a fabric softener composition as defined above is first diluted in an aqueous rinse bath solution. Subsequently, the laundered fabrics which have been washed with a detergent liquor and optionally rinsed in one or more inefficient rinse steps ("inefficient" in the sense that residual detergent and/or soil may be carried over with the fabrics) are placed in the rinse solution with the diluted composition. Of course, the fabric softener composition may also be incorporated into the aqueous bath once the fabrics have been immersed therein. Following that step, agitation is applied to the fabrics in the rinse bath solution causing the suds to collapse, and residual soils and surfactant are to be removed. The fabrics can then be optionally wrung before drying.

The non-rinse/rinse process may be performed manually in a basin or bucket, in a non-automated washing machine, or in an automated washing machine. When hand washing is performed, the laundered fabrics are removed from the detergent liquor and wrung out. The fabric softener of the present invention is then added to fresh water and the fabrics are then, directly in case of the non-rinse process or after one or more optional inefficient rinse steps in case of the rinse process, rinsed in the water containing the composition according to the conventional rinsing habit. The fabrics are then dried using conventional means.

The fabric softener composition can be used to soften fabrics by treating the fabric with the composition. This can be done during the non-rinse and rinse process using a liquid fabric softener.

The following examples are given in order to provide a person skilled in the art with a sufficiently clear and complete explanation of the present invention, but should not be considered as limiting of the essential aspects of its subject, as set out in the preceding portions of this description.

### EXAMPLES

The first part of the Examples section refers to the preparation of the fabric softener active compositions according to the invention.

The second and third part of the Examples indicates analytical methods and physical properties methods, respectively, used to analyse the prepared fabric softener active compositions.

The fourth part of the Examples section presents some fundamental physical-chemical characteristics of the prepared fabric softener active compositions: content of residual amine, content of fatty acid ester, content of free fatty acid, content of fatty alcohol, dropping point, and melt viscosity.

The fifth part of the Examples refers to the preparation of the fabric softener compositions according to the invention, the determination of the initial viscosity of the aqueous dispersions and to the performance evaluation of their softening properties. 1. Preparation of the fabric softener active compositions according to the invention. Selected examples correspond to the fabric softener active compositions.

### Comparative Example 1

### Esterification

1800 grams (6.62 mol) of tallow fatty acid and 600 grams (2.21 mol) of hydrogenated tallow fatty acid were introduced in an inert atmosphere into a stainless steel reactor, and 796.8 grams (5.35 mol) of triethanolamine were added with stirring. The mixture was heated for at least 4 hours at 160-180°C in order to remove water from the reaction. The final point of the reaction was monitored by an acid value assay, until the value was below 2 mg KOH/g.
A yellowish liquid product from the esterification step was obtained, consisting essentially of a mixture of unesterified fatty acids, mono-, di- and triesterified triethanolamine and unreacted triethanolamine.

### Quaternization

40.7 grams (0.15 mol) of tallow fatty acid and 13.6 grams (0.05 mol) of hydrogenated tallow fatty acid were added with stirring to 852 grams of the product from the esterification step (containing 1.50 mol of esteramine). Then, 179.8 grams (1.43 mol) of dimethyl sulphate were added with stirring at a temperature of 50-90°C. After four hours of digestion, the virtually complete absence of amine value was verified by acid/base assay. 1087.2 grams of the product containing the quaternized esteramine were obtained.

### Example 2

### Esterification

674.6 grams (2.48 mol) of tallow fatty acid and 224.9 grams (0.82 mol) of hydrogenated tallow fatty acid were introduced in an inert atmosphere into a stainless steel reactor, then 272.7 grams (1.83 mol) of triethanolamine were added together with 93.0 grams (0.35 mol) of stearyl alcohol with stirring. The mixture was heated for at least 4 hours at 160-180°C in order to remove water from the reaction. The final point of the reaction was monitored by an acid value assay, until the value was below 2 mg KOH/g.
A yellowish liquid product from the esterification step was obtained, consisting essentially of a mixture of unesterified fatty acids, mono-, di- and triesterified triethanolamine, esterified fatty alcohol, unreacted triethanolamine and fatty alcohol (stearyl alcohol).

### Quaternization

1174.2 grams of the product from the esterification step (containing 1.87 mol of esteramine) were reacted with 224.1 grams (1.78 mol) of dimethyl sulphate, which were added with stirring at a temperature of 50-90°C. After four hours of digestion, the virtually complete absence of amine value was verified by acid/base assay. Finally, 73.6 grams of isopropanol were added to obtain a total of 1471.8 grams of the final product.

### Example 3

### Esterification

657.4 grams (2.42 mol) of tallow fatty acid and 219.1 grams (0.81 mol) of hydrogenated tallow fatty acid were introduced in an inert atmosphere into a stainless steel reactor, then 247.5 grams (1.66 mol) of triethanolamine were added together with 189.9 grams (0.71 mol) of stearyl alcohol with stirring. The mixture was heated for at least 4 hours at 160-180°C in order to remove water from the reaction. The final point of the reaction was monitored by an acid value assay, until the value was below 2 mg KOH/g.
A yellowish liquid product from the esterification step was obtained, consisting essentially of a mixture of unesterified fatty acids, mono-, di- and triesterified triethanolamine, esterified fatty alcohol, unreacted triethanolamine and fatty alcohol (stearyl alcohol).

### Quaternization

1231.4 grams of the product from the esterification step (containing 1.71 mol of esteramine) were reacted with 204.5 grams (1.62 mol) of dimethyl sulphate, which were added with stirring at a temperature of 50-90°C. After four hours of digestion, the virtually complete absence of amine value was verified by acid/base assay. Finally, 44.4 grams of isopropanol were added to obtain a total of 1480.1 grams of the final product.

### 2. Analytical methods

### Potentiometric acid / Base titrations

Content on amine salt and free fatty acid were determined by non-aqueous potentiometric titration with KOH. Samples were dissolved in 2-propanol.

Total amine value was determined by non-aqueous potentiometric titration with perchloric acid solution in glacial acetic acid.

Residual amine value, which corresponds to the non-quaternized amine fraction, was calculated as the sum of total amine value and amine salt.

All these values are expressed as mg KOH per g.

### GLC analysis

Content of fatty acid **fatty alcohol** ester and free fatty alcohol were determined by GLC analysis, using an internal standard. Samples were dissolved in chloroform.

### 3. Physical properties methods

**Dropping point** was determined by the capillary method as the temperature at which the first drop falls or flows out of the standard cylindrical cup with a circular hole with a diameter of 2.8 mm in the bottom. Samples were melted and introduced in the cup. They were left to solidify between 12 - 24 hours in a refrigerator at a low temperature (-20°C)(an initial temperature of at least 5°C below the expected dropping point is required). Samples were then subjected to a constant heating rate (1°C/min) to the point when they flowed through the hole, corresponding to the dropping point.

**Melt viscosities** were taken at 70°C on a Rheometer Haake model RS600 at a shear rate of 5 s-1 using 60mm serrated parallel plates with a plate distance of 0.8 mm.

### 4. Physical-chemical characteristics of the prepared fabric softener active compositions.

Physical-chemical characteristics of the fabric softener active compositions, prepared as it has been described in the first part of the Examples section, are summarized in Table 1 below.

**Table 1**

| **Physical-chemical property** | **1 (comparative)** | **2** | **3** |
|---|---|---|---|
| **FA: Tallow/ Hydrogenated tallow ratio** | 75/25 | 75/25 | 75/25 |
| **Residual amine value (mg KOH/g)** | 14.6 | 7.9 | 7.4 |
| **(b) Fatty acid fatty alcohol ester (%)** | - | 7.1 | 11.5 |
| **(c) Free fatty acid (%)** | 2.1 | 0.4 | 0.4 |
| **(d) Free fatty alcohol (%)** | - | 2.0 | 5.7 |
| **(e) Isopropanol** | - | 5 | 3 |
| **Dropping point (°C)** | 64.1 | 44.0 | 47.7 |
| **Viscosity (cP) at 70°C** | 3307 | 227 | 256 |

Examples 2 to 3 correspond to fabric softener active compositions within the scope of the invention, and show dropping points below 60°C which will allow the handling at molten state at maximum 70°C, ensuring a good chemical stability. In the same way, all fabric softener active compositions according to the invention show good viscosity values at 70°C, so that they can be easily pumped in the molten state.

In contrast, example 1 (comparative) accounts for a fabric softener active composition not within the scope of the invention. It has a dropping point above 60°C, which would demand a handling temperature overcoming 70°C, compromising the chemical stability of the product. Accordingly, the fabric softener active compositions within the scope of the invention have suitable viscosity at low content or in the absence of flammable solvents and at the same time.

### 5. Preparation of the fabric softener compositions according to the invention and performance evaluation of their softening properties.

Fabric softener compositions were made by dispersing fabric softener active compositions into water.
Aqueous dispersions shown in Table 2 contain 4.5% of fabric softener active compositions and 0.1% active of a thickening polymer (i.e. FLOSOFT 222 manufactured by SNF).

The dispersion process consists of heating deionized water at 60 °C in a jacketed glass reactor, adding the thickening polymer while stirring until complete incorporation, adding the fabric softener active composition of interest in the molten state (heated 5 to 10°C above the melting point) and homogenizing the dispersion at a rate of 150 rpm during 20 min. The aqueous dispersion is finally cooled down up to 25-30°C, at rate of 1.0°C/min, maintaining the agitation at 150 rpm.

Initial viscosity of the aqueous dispersions was determined at 20°C, 24 h after preparation, with a Brookfield viscosimeter model LV, using a spindle number 2 at 60rpm.

Softening performance of fabric softener compositions was determined by means of a sensorial test carried out by a panel of experts using pieces of terry cotton towel treated with the corresponding aqueous dispersions of the fabric softener active compositions.

Fabric treatment consists of a consecutive sequence of washing and softening steps, carried out in hard water of 20°HF. Previously scoured terry cotton towels were washed at 40°C with a heavy duty powder detergent (at a dosage of 2.7% on weight fabric), rinsed twice and spinning dried. Wet towels were treated for 10 minutes at 25°C with the corresponding aqueous dispersions diluted in water to provide a dosage of 0.12% fabric softener active composition on weight fabric, for a bath ratio of 1/10. Treated cotton towels were finally spun dried and let dry by hanging, and left still for 24 hours under controlled atmospheric conditions (60%HR and 20°C).
Softening effect was determined by comparison in pairs, by 12 panelists, against standard products of equivalent hydrogenation degree (comparative examples C1 and C2). Results are indicated in Table 2.The comparative evaluation was made according to the following criteria:
+ 2: softer than the reference
+1: slightly softer than the reference
0 as soft as the reference
-1: slightly harder than the reference
-2: harder than the reference

**Table 2**

| **Fabric softener active composition** | **2** | **3** | **4** | **C1** | **C2** |
|---|---|---|---|---|---|
| **Initial viscosity at 20°C (cP)** | **40** | **55** | **140** | **90** | **115** |
| **Softening effect** | **+2** | **+2** | **+1** | **-** | **-** |
| **compared to** | **C1** | **C1** | **C1** | **-** | **-** |

### C1: TETRANYL®L1/90 available from Kao Corporation, Ester Quat with a tallow/ hydrogenated tallow ratio of 75/25

It can be seen that all fabric softener active compositions within the scope of the invention provide acceptable viscosity values and higher softening effects than the fabric softener active compositions of the corresponding comparative examples.

## Claims

1. A fabric softener active composition comprising:
- a component (a), said component comprising at least one or more quaternary mono-, di- or tri-ester ammonium compounds;
- a component (b), said component being a fatty acid ester or a mixture of fatty acid esters derived from a C₁₈-C₂₀ fatty alcohol, wherein the component (b) content is in the range from 5 to 60% wt. based on the total weight of the fabric softener active composition;
- a component (c), said component being a fatty acid or a mixture of fatty acids, wherein the component (c) content is higher than 0 and lower than 5, preferably lower than 2% wt. based on the total weight of the fabric softener active composition;
- a component (d), said component being a C₁₈-C₂₀ fatty alcohol, preferably a C₁₈ fatty alcohol; and wherein the component (d) content is in the range from 2 to 12% wt. based on the total weight of the fabric softener active composition.

2. The fabric softener active composition according to claim 1 further comprising a component (e), said component being a solvent, wherein the component (e) content is higher than 0% wt. and lower than 8% wt. based on the total weight of the fabric softener active composition; or alternatively, in the absence of the component (e).

3. The fabric softener active composition according to claim 1 or 2 wherein the component (b) is a fatty acid ester or a mixture of fatty acid esters derived from a C₁₈ fatty alcohol.

4. The fabric softener active composition according to any one of the claims 1-3 wherein the component (c) is a C₈-C₂₀ fatty acid or a mixture of C₈-C₂₀ fatty acids, preferably a C₁₂-C₂₀ fatty acid or a mixture of C₁₂-C₂₀ fatty acids.

5. The fabric softener active composition according to any one of the claims 1-4 wherein the component (a), the component (b), and the component (c) are derived from the same fatty acid or mixture of fatty acids.

6. A method for producing a fabric softener active composition as defined in any of the claims 1-5 comprising:
i) an esterification step, wherein a fatty acid, a methyl ester or a triglyceride thereof is reacted with an alkanolamine to obtain a mixture containing an esteramine; and
ii) a quaternization step, wherein the mixture obtained after the esterification step is reacted with an alkylating agent.

7. The method according to claim 6, wherein the component (b) present in the fabric softener active composition is intentionally added and/or generated in situ in the esterification step, after the esterification step, in the quaternization step or after the quaternization step.

8. The method according to any of the claims 6-7, wherein the component (b) is generated in situ by reaction with component (d) which is added in the esterification step, after the esterification step, in the quaternization step or after the quaternization step .

9. The method according to any of the claims 6-8, wherein component (c) present in the fabric softener active composition is intentionally added in the esterification step, after the esterification step, in the quaternization step or after the quaternization step.

10. A fabric softener composition comprising a fabric softener active composition as defined in any of the claims 1-5, further comprising at least water, wherein the fabric softener active composition is present in an amount from 1 to 30% wt. based on the total weight of the fabric softener composition, and optionally comprising the further components:
- from 0 to 2% of an electrolyte concentration aid; and/or
- from 0.01 to 3% of a thickening polymer; and/or
- from 0.01 to 5% of a perfume.

11. Use of the fabric softener composition as defined in claim 10 to soften and condition fabrics.

12. A method for producing the fabric softener composition as defined in the claim 10, comprising the steps:
i) adding the fabric softener active composition as defined in any one of claims 1-5 in a molten state to water;
ii) stirring to obtain a homogeneous dispersion.

13. The method according to claim 12, further comprising the steps:
iii) cooling down; and
iv) optionally mixing with the further components, wherein the further components can be added to the aqueous phase before or after the dispersion of the fabric softener active ingredient, and before or after any one of steps i) to iii).

## Patentansprüche

1. Weichspülerwirkstoffzusammensetzung, umfassend:
- eine Komponente (a), wobei diese Komponente mindestens eine oder mehr quaternäre Mono-, Di- oder Triesterammoniumverbindungen enthält;
- eine Komponente (b), wobei diese Komponente ein Fettsäureester oder eine Mischung von Fettsäureestern, abgeleitet von einem C₁₈₋₂₀-Fettalkohol, ist, wobei der Gehalt der Komponente (b) im Bereich von 5 bis 60 Gew.-% liegt, basierend auf dem Gesamtgewicht der Weichspülerwirkstoffzusammensetzung;
- eine Komponente (c), wobei diese Komponente eine Fettsäure oder eine Mischung von Fettsäuren ist, wobei der Gehalt der Komponente (c) höher als 0 und niedriger als 5, vorzugsweise niedriger als 2 Gew.-% ist, basierend auf dem Gesamtgewicht der Weichspülerwirkstoffzusammensetzung;
- eine Komponente (d), wobei diese Komponente ein C₁₈₋₂₀-Fettalkohol, vorzugsweise ein C₁₈-Fettalkohol, ist; und wobei der Gehalt der Komponente (d) im Bereich von 2 bis 12 Gew.-% liegt, basierend auf dem Gesamtgewicht der Weichspülerwirkstoffzusammensetzung.

2. Weichspülerwirkstoffzusammensetzung gemäß Anspruch 1, weiterhin umfassend eine Komponente (e), wobei diese Komponente ein Lösungsmittel ist, wobei der Gehalt der Komponente (e) höher als 0 Gew.-% und niedriger als 8 Gew.-% ist, basierend auf dem Gesamtgewicht der Weichspülerwirkstoffzusammensetzung; oder alternativ, in der Abwesenheit der Komponente (e).

3. Weichspülerwirkstoffzusammensetzung gemäß Anspruch 1 oder 2, wobei die Komponente (b) ein Fettsäureester oder eine Mischung von Fettsäureestern, abgeleitet von einem C₁₈-Fettalkohol, ist.

4. Weichspülerwirkstoffzusammensetzung gemäß einem der Ansprüche 1-3, wobei die Komponente (c) eine C₈₋₂₀-Fettsäure oder eine Mischung von C₈₋₂₀-Fettsäuren, vorzugsweise eine C₁₂₋₂₀-Fettsäure oder eine Mischung von C₁₂₋₂₀-Fettsäuren, ist.

5. Weichspülerwirkstoffzusammensetzung gemäß einem der Ansprüche 1-4, wobei die Komponente (a), die Komponente (b) und die Komponente (c) von der gleichen Fettsäure oder Mischung von Fettsäuren abgeleitet sind.

6. Verfahren zum Herstellen einer Weichspülerwirkstoffzusammensetzung, definiert wie in einem der Ansprüche 1-5, umfassend:
i) einen Veresterungsschritt, worin eine Fettsäure, ein Methylester oder ein Triglycerid davon mit einem Alkanolamin reagieren gelassen wird, so dass eine Mischung, die ein Esteramin enthält, erhalten wird; und
ii) einen Quaternisierungsschritt, worin die nach dem Veresterungsschritt erhaltene Mischung mit einem Alkylierungsmittel reagieren gelassen wird.

7. Verfahren gemäß Anspruch 6, wobei die in der Weichspülerwirkstoffzusammensetzung vorhandene Komponente (b) im Veresterungsschritt, nach dem Veresterungsschritt, im Quaternisierungsschritt oder nach dem Quaternisierungsschritt gezielt zugegeben und/oder in situ generiert wird.

8. Verfahren gemäß einem der Ansprüche 6-7, wobei die Komponente (b) in situ generiert wird durch Reaktion mit Komponente (d), die im Veresterungsschritt, nach dem Veresterungsschritt, im Quaternisierungsschritt oder nach dem Quaternisierungsschritt zugegeben wird.

9. Verfahren gemäß einem der Ansprüche 6-8, wobei die in der Weichspülerwirkstoffzusammensetzung vorhandene Komponente (c) im Veresterungsschritt, nach dem Veresterungsschritt, im Quaternisierungsschritt oder nach dem Quaternisierungsschritt gezielt zugegeben wird.

10. Weichspülerzusammensetzung, umfassend eine Weichspülerwirkstoffzusammensetzung, definiert wie in einem der Ansprüche 1-5, weiterhin umfassend Wasser, wobei die Weichspülerwirkstoffzusammensetzung in einem Gehalt von 1 bis 30 Gew.-% vorliegt, basierend auf dem Gesamtgewicht der Weichspülerzusammensetzung und optional umfassend die weiteren Komponenten:
- von 0 bis 2 % einer Elektrolytkonzentrationshilfe; und/oder
- von 0,01 bis 3 % eines Verdickerpolymers; und/oder
- von 0,01 bis 5 % eines Parfüms.

11. Verwendung der Weichspülerzusammensetzung, definiert wie im Anspruch 10, zum Weichspülen und Konditionieren von Textilien.

12. Verfahren zum Herstellen der Weichspülerzusammensetzung, definiert wie im Anspruch 10, umfassend die Schritte:
i) Zugeben der Weichspülerwirkstoffzusammensetzung, definiert wie in einem der Ansprüche 1-5, in geschmolzenem Zustand zu Wasser;
ii) Rühren, so dass eine homogene Dispersion erhalten wird.

13. Verfahren gemäß Anspruch 12, weiterhin umfassend die Schritte:
ii) Abkühlen; und
iii) optional Mischen mit den weiteren Komponenten, wobei die weiteren Komponenten vor oder nach der Dispersion des Weichspülerwirkstoffbestandteils und vor oder nach einem beliebigen der Schritt i) bis iii) zur wässrigen Phase zugegeben werden können.

## Revendications

1. Composition active d'assouplissant pour textile comprenant :
- un composant (a), ledit composant comprenant au moins un ou plusieurs composés d'ammonium quaternaire à mono-, di- ou tri-ester ;
- un composant (b), ledit composant étant un ester d'acide gras ou un mélange d'esters d'acide gras dérivés d'un alcool gras en C₁₈ à C₂₀, dans laquelle la teneur en composant (b) est située dans la plage allant de 5 à 60 % en poids sur la base du poids total de la composition active d'assouplissant pour textile ;
- un composant (c), ledit composant étant un acide gras ou un mélange d'acides gras, dans laquelle la teneur en composant (c) est supérieure à 0 et inférieure à 5, de préférence inférieure à 2 % en poids sur la base du poids total de la composition active d'assouplissant pour textile ;
- un composant (d), ledit composant étant un alcool gras en C₁₈ à C₂₀, de préférence un alcool gras en C₁₈ ; et dans laquelle la teneur en composant (d) est située dans la plage allant de 2 à 12 % en poids sur la base du poids total de la composition active d'assouplissant pour textile.

2. Composition active d'assouplissant pour textile selon la revendication 1, comprenant en outre un composant (e), ledit composant étant un solvant, dans laquelle la teneur en composant (e) est supérieure à 0 % en poids et inférieure à 8 % en poids sur la base du poids total de la composition active d'assouplissant pour textile ; ou en variante, en l'absence du composant (e).

3. Composition active d'assouplissant pour textile selon la revendication 1 ou 2, dans laquelle le composant (b) est un ester d'acide gras ou un mélange d'esters d'acide gras dérivés d'un alcool gras en C₁₈.

4. Composition active d'assouplissant pour textile selon l'une quelconque des revendications 1 à 3, dans laquelle le composant (c) est un acide gras en C₈ à C₂₀ ou un mélange d'acides gras en C₈ à C₂₀, de préférence un acide gras en C₁₂ à C₂₀ ou un mélange d'acides gras en C₁₂ à C₂₀.

5. Composition active d'assouplissant pour textile selon l'une quelconque des revendications 1 à 4, dans laquelle le composant (a), le composant (b) et le composant (c) sont dérivés du même acide gras ou mélange d'acides gras.

6. Procédé de production d'une composition active d'assouplissant pour textile telle que définie dans l'une quelconque des revendications 1 à 5, comprenant :
i) une étape d'estérification, dans lequel un acide gras, un ester de méthyle ou un triglycéride de celui-ci est mis à réagir avec une alcanolamine pour obtenir un mélange contenant une esteramine ; et
ii) une étape de quaternisation, dans lequel le mélange obtenu après l'étape d'estérification est mis à réagir avec un agent d'alkylation.

7. Procédé selon la revendication 6, dans lequel le composant (b) présent dans la composition active d'assouplissant pour textile est intentionnellement ajouté et/ou généré in situ dans l'étape d'estérification, après l'étape d'estérification, dans l'étape de quaternisation ou après l'étape de quaternisation.

8. Procédé selon l'une quelconque des revendications 6 à 7, dans lequel le composant (b) est généré in situ par réaction avec le composant (d) qui est ajouté dans l'étape d'estérification, après l'étape d'estérification, dans l'étape de quaternisation ou après l'étape de quaternisation.

9. Procédé selon l'une quelconque des revendications 6 à 8, dans lequel le composant (c) présent dans la composition active d'assouplissant pour textile est ajouté intentionnellement dans l'étape d'estérification, après l'étape d'estérification, dans l'étape de quaternisation ou après l'étape de quaternisation.

10. Composition d'assouplissant pour textile comprenant une composition active d'assouplissant pour textile telle que définie dans l'une quelconque des revendications 1 à 5, comprenant en outre au moins de l'eau, dans laquelle la composition active d'assouplissant pour textile est présente en une quantité de 1 à 30 % en poids sur la base du poids total de la composition d'assouplissant pour textile, et comprenant facultativement les composants supplémentaires :
- de 0 à 2 % d'un auxiliaire de concentration d'électrolyte ; et/ou
- de 0,01 à 3 % d'un polymère épaississant ; et/ou
- de 0,01 à 5 % d'un parfum.

11. Utilisation de la composition d'assouplissant pour textile telle que définie dans la revendication 10 pour assouplir et conditionner les textiles.

12. Procédé de production de la composition d'assouplissant pour textile telle que définie dans la revendication 10, comprenant les étapes suivantes :
i) l'ajout de la composition active d'assouplissant pour textile telle que définie dans l'une quelconque des revendications 1 à 5 dans un état fondu à de l'eau ;
ii) l'agitation pour obtenir une dispersion homogène.

13. Procédé selon la revendication 12, comprenant en outre les étapes suivantes :
iii) le refroidissement ; et
iv) facultativement le mélange avec les composants supplémentaires, dans lequel les composants supplémentaires peuvent être ajoutés à la phase aqueuse avant ou après la dispersion de l'ingrédient actif d'assouplissant pour textile, et avant ou après l'une quelconque des étapes i) à iii).
